Europäisches Patentamt

European Patent Office

Office européen des brevets

(19)

(11) Publication number: **0 175 270**
**B1**

(12) EUROPEAN PATENT SPECIFICATION

(45) Date of publication of patent specification: **17.11.88**

(21) Application number: **85111469.4**

(22) Date of filing: **11.09.85**

(51) Int. Cl.⁴: **G 01 N 33/564**

(54) Prognostic value of rheumatoid factor isotypes and their association with disease activity.

(30) Priority: **19.09.84 US 652072**
**19.09.84 US 652073**

(43) Date of publication of application:
**26.03.86 Bulletin 86/13**

(45) Publication of the grant of the patent:
**17.11.88 Bulletin 88/46**

(84) Designated Contracting States:
**DE FR GB IT SE**

(56) References cited:
**DD-A- 230 940**
**DE-A-2 707 881**
**GB-A-2 114 289**
**US-A-4 292 403**

**CLINICAL CHEMISTRY, vol. 30, no. 6, June 1984, pages 880-883, Washington, US; I. GIAEVER et al.: "A new assay for rheumatoid factor"**
**ARTHRITIS AND RHEUMATISM, vol. 24, no. 12, December 1981, pages 1501-1511, US; R. WERNICK et al.: "Serum IgG and IgM rheumatoid factors by solid phase radioimmunoassay"**

(73) Proprietor: **Valdimarsson, Helgi**
**Laekjaras 16**
**110 Reykjavik (IS)**
(73) Proprietor: **Teitsson, Ingvar**
**114 Simshill Road**
**Glasgow G44 5EN (GB)**

(72) Inventor: **Valdimarsson, Helgi**
**Laekjaras 16**
**110 Reykjavik (IS)**
Inventor: **Teitsson, Ingvar**
**114 Simshill Road**
**Glasgow G44 5EN (GB)**

(74) Representative: **De Minvielle-Devaux, Ian Benedict Peter et al**
**CARPMAELS & RANSFORD 43, Bloomsbury Square**
**London WC1A 2RA (GB)**

EP 0 175 270 B1

**Description**

Rheumatoid factors (RF) of the agglutinating type occur in over 75% of rheumatoid arthritis (RA) patients and intracellular IgG RF complexes have been reported to be particularly prominent in synovial tissues from sero-negative RA patients. Thus, RFs are an immunological hallmark of RA and may be directly involved in the pathogenesis. There is considerable uncertainty, however, as to the relationship between rheumatoid factors and clinical dieseas activity. Otten and Boerma [*Ann. Rheum. Dis. 18*: 24—28 (1959)] found no close association between levels of agglutinating RF and disease activity and most authors are in agreement. Agglutinating RFs are predominantly of the IgM isotype but IgG RF and IgA RF are also known to occur and recent progress in solid phase immunoassay techniques has enabled the development of isotype-specific RF assays.

Rheumatoid arthritis may vary in severity from one mild attack to progressive, destructive disease with early death from systematic complications. There is now substantial evidence that treatment with "specific" drugs like gold salts may favorably alter the course of RA. However, toxic reactions occur in 20—40% of patients treated wqth gold or penicillamine and since these reactions may occasionally be life-threatening, the "specific" drugs tend to be reserved for those with more severe disease. An early identification of patients with a poor prognosis is important because there is evidence that "specific" treatment is most effective early in the course of RA. Several workers have studied patients with early RA prospectively in an attempt to identify features of prognostic importance. Accurate prognostication in early RA is difficult but slow onset and strongly positive agglutination tests for rheumatoid factor (RF) are generally thought to imply poor prognosis [*Am. Rheum. Dis. 23*: 193—204 (1964); *Ann. Rheum. Dis. 18*: 24—28 (1959); *Br. Med. J. 2*: 96—100 (1973); and *Ann. Rheum. Dis. 35*; 357—360 (1976)].

Agglutination tests primarily detect IgM antibodies as other antibodies are poor agglutinators. Rheumatoid factors of isotypes other than IgM do exist, however, and may be of fundamental importance in the pathogenesis of RA. The main goals of the present study were to assess the clinical value of measuring individual RF isotypes in early RA and to define more closely the relationship between fluctuations in levels of individual RF isotypes and clinical indices of disease activity. The results indicate that the raised serum IgA RF is reliable early marker of adverse prognosis in RA. The results further indicate that serum IgA RF levels reflect fluctuations in disease activity more closely than the levels of IgG RF or IgM RF.

The present application relates to a method for aiding the prognosis of individuals afflicted with rheumatoid arthritis, characterized in that:

(a) the level of the IgA isotype of rheumatoid factor (RF) in the blood of such individuals is measured, and

(b) such measured IgA RF level is compared with the level of normal individuals, whereby a substantially increased level of IgA RF in the tested individual indicates a likelihood that the disease will proceed to a severe state with the appearance of bone erosions.

The present applicatugn also relates to a method for aiding the prognosis of individuals afflicted with rheumatoid arthritis characterized in that:

(a) the level of the IgM isotype of rheumatoid factor (RF) in the blood of such individuals is measured, and

(b) such measured IgM RF level is compared with the level of normal individuals, whereby a substantially increased level of IgM RF in the tested individual indicates a likelihood that the disease will not proceed to a severe state.

Materials and methods
A. Prognostic value of IgA RF
Rheumatoid factor assays

Enzyme-linked immunosorbent assays (ELISA) were developed for the measurement of rheumatoid factors of IgM, IgA and IgG isotypes in serum and synovial fluid [Teitsson, "Antiglobulins in relation to rheumatic diseases: development of ELISA assays and their application in prospective and family surveys". University of London, Ph. D. thesis (1983)]. Flat-well microplates (M29AR®, Sterilin Ltd., UK) were coated with a 10 µg/ml solution of normal rabbit IgG and saturated with 1% bovine serum albumin (BSA). Subsequently, the plates were incubated with samples diluted in PBS containing 0.5M sodium chloride and 0.5% Tween® 20. Sample dilutions used were 1/10, 1/100 and 1/1000 for IgM RF and IgA RF. Samples were diluted 1/10 only for the IgG RF assay because of low reactivity at the higher dilutions. Prior to the IgG RF assay samples were digested with pepsin (Cat. No. P-7012, Sigma London) as described by Wernick et al, *Arthritis Rheum. 24*: 1501—1511 (1981).

Subsequently, the plates were incubated with dilutions of anti-human immunoglobulins, labelled with alkaline phosphatase (AP) (type VII-S, Sigma London) as described by Voller et al, *Bull. WHO 53*: 55065 (1976), Affinity-purified goat anti-human IgM (Code No. 4102, TAGO Inc., USA) was used for the IgM RF assay but the F(ab')₂ fraction of a rabbit anti-human IgA antibody was used for the IgA RF assay.

In the IgG RF assay, sample incubation was followed by incubation with an unlabelled monoclonal mouse anti-human Fab-gamma (Code No. 63-070, Miles Laboratories, Ltd.). Subsequently, the plates were incubated with the AP conjugated F(ab')₂ fraction of a goat anti-mouse IgG (Code No. 4250, TAGO, Inc.,

USA). Unlabelled mouse antibody was used because IgM RF was found to react poorly with mouse IgG, and glutaraldehyde-aggregated IgG antibody may also show enhanced reactivity with IgM RF, yielding false-positive results in the presence of IgM RF.

Finally, the plates were reacted with a 1 mg/ml solution of p-nitrophenyl phosphate and read at 405 nm in a Titertek® Multiskan microplate reader (Flow Laboratories).

Expression and standardisation of RF results

Each sample dilution was incubated in one uncoated and three adjacent IgG-coated wells. The mean net absorbance for each dilution and the combined net absorbance (CNA) for the three dilutions in the IgA RF and IgM RF assays were calculated. The mean net absorbance (IgG RF) and CNA values were read against dilution curves of internal standards and the results expressed in units (U) of RF per ml. The internal standards were three rheumatoid sera which reacted exceptionally strongly in the respective RF assays. The IgM RF standard was calibrated against the "1st British Standard for Rheumatoid Arthritis Serum" [*Bull WHO 42*: 311—318 (1970)] and found to contain 875 international units (IU) per ml. The IgA RF and IgG RF standars were each assigned an arbitrary value of 1000 U/ml.

Pepsin digestion of the IgA RF standard was found to abolish its IgM RF activity without affecting the IgA RF level significantly.

Sera from 102 healthy Caucasians (age range 7—80 years) were assaysd for IgM, RF, IgA RF and IgG RF in order to establish the upper limit of normal for each RF isotype.

Patients studied

Thirty-three patients with early inflammatory joint disease of less than one year's duration at presentation were followed prospectively for 2—4 years. These patients who are described in more detail below presented with a peripheral synovitis suggestive of RA. The follow-up included 3-monthly assessments of early morning stiffness (EMS), global pain using a horizontal visual analogue scale (PVAS), the Ritchie articular index (RAI), grip strength (GS) and erythrocyte sedimentation rate (ESR). The overall index of disease activity for each visit (IDA) was calculated from these five parameters.

Serum and synovial fluid samples were collected during the follow-up, stored at −70°C and assayed for the three RF isotypes at the end of the study. All samples from each patient were assayed on the same day in order to reduce errors due to day-to-day fluctuations in the assays.

Hand and wrist X-rays were taken at the time of entry to the study and annually thereafter. The serial films for each patient were assessed, without knowledge of the identity or clinical featurs, in order to determine the total defect score (erosions) for each film [*Arthritis Rheum. 14*: 706—720 (1971)].

Statistical analysis

The relationship between the clinical features and the final erosion count was assessed by the Spearman's rank correlation method. Wilcoson's two-sample test, chi-square test with Yate's correction and Fisher's exact probability test were used to assess the difference in outcome between those with raised and normal initial RF levels.

B. Association with disease activity

Patients studied

Patients were admitted to the study if, on referral to the Rheumatology Outpatient Department, they had active peripheral synovitis, suggestive of RA of less than one year's duration. Those who had already been treated with gold, penicillamine, corticosteroids or cytotoxic drugs were excluded. Patients with evidence of gout, ankylosing spondylitis, Reiter's disease, psoriatic arthritis or colitic arthropathy were also excluded.

Follow-up, assessment protocol

The patients were followed in a special outpatient clinical which was held at the same time of day throughout the study. They were assessed every three months according to a protocol, and seen more frequently if necessary because of therapy. The same physician performed all the clinical assessments. If, after a minimum of 2 year follow-up, the patient was asymptomatic, the interval between assessments was sometimes extended to 6 months.

The protocol included the following:

(a) duration in minutes of early morning stiffness (EMS).

(b) global pain where the patient marked the severity of his/her overall pain on a 10 cm long horizontal visual analogue scale (PVAS).

(c) overall joint tenderness, expressed as the Ritchie articular index (RAI), by the method of Ritchie et al [*Q. J. Med. 37*: 393—406 (1968)].

(d) grip strength (GS) measured by a specially designed grip bag [*Proc. Roy. Soc. Med. 59* (Suppl): 85—88 (1966)].

(e) blood was drawn at each assessment for estimation of erythrocyte sedimentation rate (ESR) and RF levels, using both conventional agglutination tests (RAHA and latex) and isotype-specific ELISA. Those patients whose serum was RAHA positive (titre≥1/40) at least one were considered to meet the 8th ARA criterion for seropositivity [*Bull. Rheum. Dis. 9*: 175—176 1958)].

Data analysis

The overall index of disease activity (IDA) was calculated for each visit. The EMS, PVAS, RAI, GS and ESR were each assigned a grade of 1—4 using the table of Mallya and Mace [*Rheumatol. Rehabil. 20*: 1417 (1981)] where grade 1 indicates normal state and grade 4 extreme affection by disease. The mean of the five constituted the IDA (range 1.0—4.0).

The results of the ELISa tests for IgM RF, IgA RF and IgG RF were expressed as units (U)/ml as described above. The clinical/serological follow-up data for each patient were analysed separately, using Speaman's rank correlation method. The coefficients ($r_s$) for the inter-parameter correlations were calculated, using a BMDP statistical package (University of California, Los Angeles, USA). Subsequently, the distribution of the coefficients was normalised by dividing each coefficient by its approximate standard deviation: $1/\sqrt{n-1}$, where n was the number of clinical assessments for each patient. The association between the individual parameters in the group of 33 was then assessed by testing the hypothesis that the means of $r_s\sqrt{n-1}$ for any two parameters was not different from zero. Student's t test was used for this purpose.

Results

The RF assays: association with RAHA, normal range

When comparing the IgM RF ELISA with those of the RAHA sheep cell agglutination test [*Med. Lab. Technol. 31*: 323—326 (1974)], it was found that the CNA at dilutions 1/10+1/100+1/1000 correlated more closely with RAHA than the net absorbance at any single dilution. Consequently, samples were routinely diluted 1/10 and 1/100 and 1/1000 in the IgM RF assay in order to obtain better quantitation and the same dilutions were used for IgA RF.

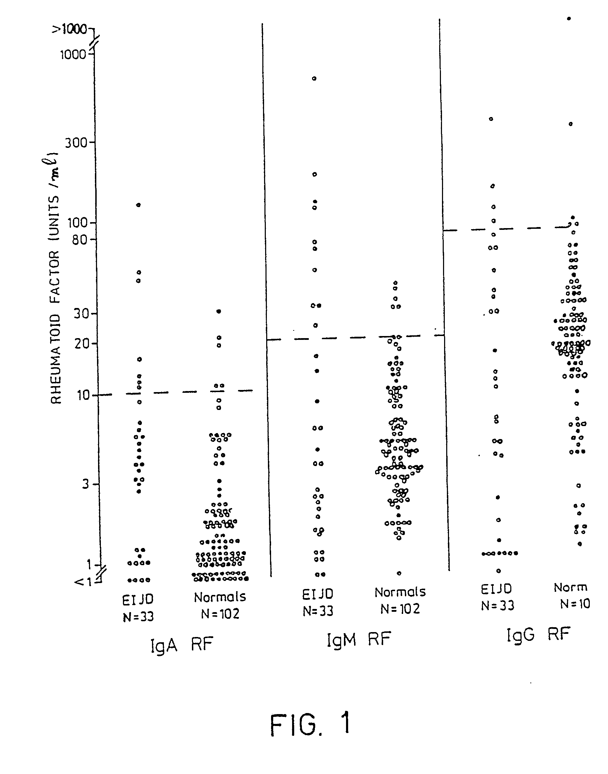

Figure 1 shows the RF levels in the 102 normal sera. By defining the 95% level as the upper limit of normal, levels >20 IU IgM RF/ml, ≥10 U IgA RF/ml and ≥80 U IgG RF/ml were considered raised.

The relationship between the clinical/serological presentation and hand and wrist erosions

Figure 1 shows the RF levels at first visit, 4.7±3.0 months (mean±1 SD) after the onset of symptoms, in the 33 patients of the prospective study group. Ten, seven and four patients had raised levels of IgM RF, IgA RF and IgG RF, respectively.

Table 1 shows the association between the final erosion count of the hands and wrists of the 33 patients and the clinical features at presentation. The initial grip strength, ESR and the index of disease activity (IDA) correlated significantly with the erosion counts (p<0.01).

TABLE 1

The association in the group of 33 between the final erosion count of the hands and wrists and clinical features at first and last visit.

|  | $r_s$* | p< |
|---|---|---|
| Age on onset | 0.350 | 0.05 |
| EMS on onset | 0.384 | 0.05 |
| PVAS at onset | 0.021 | NS |
| RAI at onset | 0.412 | 0.02 |
| GS at onset | −0.547 | 0.01 |
| ESR at onset | 0.472 | 0.01 |
| IDA at onset | 0.487 | 0.01 |
| RAI at last visit | 0.482 | 0.01 |
| GS at last visit | −0.684 | 0.001 |
| IDA at last visit | 0.544 | 0.01 |

*$r_s$: Spearman's rank correlation coefficient.

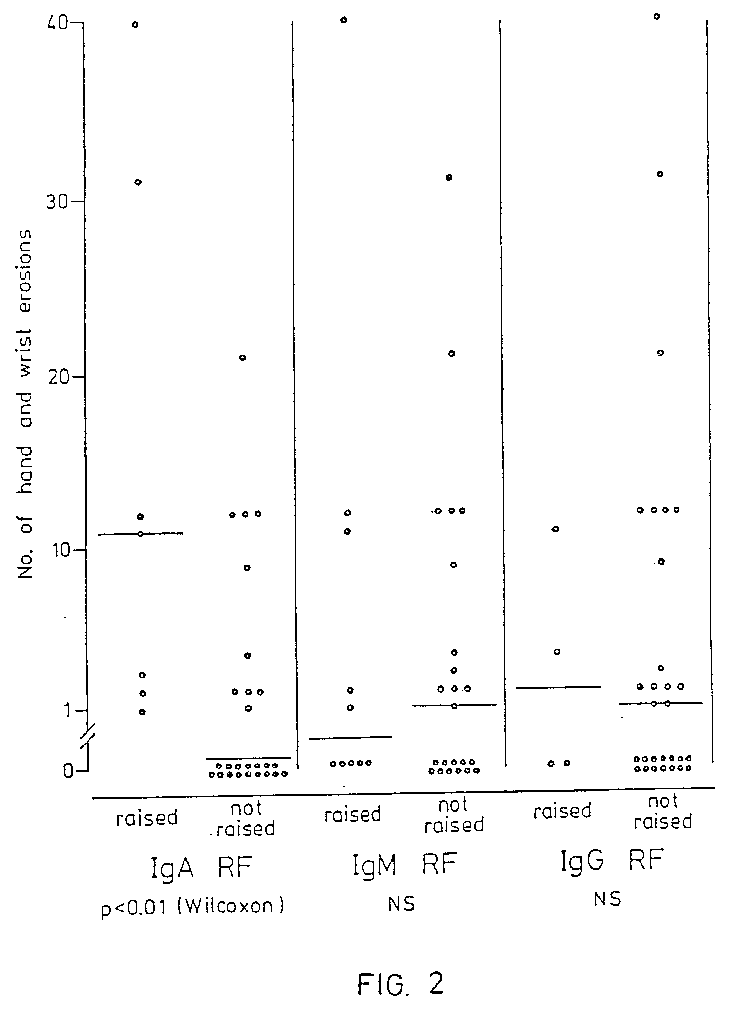

Figure 2 shows the relationship of the radio-logical outcome to the RF levels at presentation. Those seven who presented with raised levels of IgA RF all developed erosive disease and as a group they developed a significantly greater number of erosions than the remaining 26 (median erosion counts of 11 and 0, respectively, p <0.01, Wilcoxon's two-sample test). The five patients who never met the ARA criteria for classical/definite RA all presented with normal IgA RF. When these five patients were eliminated from the analysis, the difference in erosion counts was still significant (p <0.02, Wilcoxon's two-sample test). By contract, those 10 who presented the raised serum IgM RF did not develop more erosions than the remaining 23 (median erosion counts of 0.5 and 1, respectively). Similarly, those four who presented with raised IgG RD did not faire significantly worse than those with normal IgG RF (median erosion counts of 2 and 1, respectively).

Five of those who presented with raised IgA RF also had raised serum IgM RF. However, Table 2 shows that the five patients who presented with a selective increase in IgM RF remained free of erosions, in striking contrast to those seven who presented with raised IgA RF with/without raised IgM RF, as the latter group developed erosive disease without exception. This difference was statistically significant (p=0.0013, Fisher's exact probability test).

TABLE 2

The relationship of erosive hand and wrist disease to an isolated rise
in IgM RF and raised IgA RF at the first visit.

|  | Hand and wrist disease (No. of patients) | | |
|  | erosive | non-erosive | p* |
| --- | --- | --- | --- |
| Raised IgM RF with normal IgA RF | 0 | 5 | |
| Raised IgA RF with/without raised IgM RF | 7 | 0 | p=0.0013 |

*p: Fisher's exact probability test

Association between RF levels at onset and disease activity at the end of the study

Those seven patients who presented with raised IgA RF had significantly higher ESR and poorer grip strength than the rest of the group at the end of the study, see Table 3. However, there was no such association between raised IgM RF or IgG RF at onset and disease activity at the last visit.

TABLE 3

The relationship between IgA RF levels at first visit
and the grip strength and ESR at the last visit.

|  | IgA RF (onset) | Number of patients | Median | p<* |
| --- | --- | --- | --- | --- |
| Final GS (mm Hg) | ≥10 U/ml | 7 | 93 | |
|  | <10 U/ml | 26 | 211 | 0.05 |
| Final ESR (mm/h) | ≥10 U/ml | 7 | 31 | |
|  | <10 U/ml | 26 | 15 | 0.05 |

*Wilcoxon's two-sample test

Association between RF levels at first visit and subsequent treatment

Fourteen of the 28 RA patients in the prospective group were treated with gold (sodium aurothiomalate), penicillamine or oral prednisolone during the follow-up. Table 4 shows the relationship between raised/normal IgG RF at first visit and the subsequent requirement for gold, penicillamine and prednisolone. By assuming that we had a choice of these three drugs for each patient, the number of treatment opportunities for n patients was 3n. Thus defined, the seven patients who presented with raised IgA RF required significantly more drugs than the remaining 26 (p<0.001, $X^2$=17.0, chi-square test with Yates's correction) and this difference remained significant when the five non-RA patients were excluded (p<0.001, $X^2$=12.1).

TABLE 4
The relationship between IgA RF levels at onset and the
subsequent requirement for gold, penicillamine and steroids.

| IgA RF level (first visit) | No. of patients | Treatment opportunities* | | Significance** |
| --- | --- | --- | --- | --- |
| | | Used | Not used | |
| ≥10 U/ml | 7 | 14 | 7 | $X^2 = 17.0$ |
| <10 U/ml | 26 | 14 | 64 | $p < 0.001$ |

\* Each patient might have been treated with any or all of gold, penicillamine or oral prednisolone.
\*\* Chi-square test with Yates's correction

The patients studied: clinical features and treatment

Forty-six patients entered the study. Of these, 33 were followed for a minimum of 24 months from the onset of their symptoms. Of the remaining 13, who did not complete a two year follow-up, two died (one from lung cancer, the other from myocardial infarction), three moved out of the UK, and eight were lost to follow-up. Six of these eight had no joint symptoms when last seen. Unless otherwise stated, the following analysis is based exclusively on the 33 patients, followed for ≥2 years.

Table 5 shows the age and sex distribution of the group. There were 21 females and 12 males, a female/male ratio of 1.8. The mean age at the onset of symptoms was 52.9 years, age range 22—80 years. Twenty-eight patients met five or more of the ARA criteria for (RA) at some stage during the follow-up. These were classified as RA (classifical/definite) and the remaining five as non-RA (Table 5).

Seventeen of the 28 RA patients (61%) developed erosive disease of their hands and wrists during the follow-up (Table 5). The time from the onset of symptoms until the data of the last X-ray ranged from 24—28 months. However, there was no significant correlation between the duration of disease and the number of erosions in the RA group ($p > 0.05$, $r = 0.37$, Pearson's product-moment correlation method).

Eight of the RA patients required gold (sodium aurothiomalate) treatment, and Table 5 shows that six of these eight also required penicillamine and one azathioprine during the follow-up. All these eight and six other RA patients, 50% of the RA group, were treated with oral steroids (low dose prednisolone) at some stage during the follow-up. The decision to treat with the "specific" drugs or steroids was based on conventional criteria, such as persistent synovitis with disabling symptoms despite full treatment with non-steroidal anti-inflammatory drugs and progression of bone erosions on X-ray.

TABLE 5
Summary of clinical features and drug requirements of the group
of 33 who were followed for at least two years

|  | RA | Non-RA |
| --- | --- | --- |
| Total number | 28 | 5 |
| Females/males | 19/9 | 2/3 |
| Mean age at onset±1 SD | 55.9±16.5 y | 35.8±9.3 y |
| Age range at onset | 22—80 y | 26—52 y |
| No. of ARA criteria for RA: 4 | — | 5 |
| No. of ARA criteria for RA: 5 | 7 | — |
| No. of ARA criteria for RA: 6 | 13 | — |
| No. of ARA criteria for RA: 7 | 8 | — |
| Erosive disease (hands and wrists) | 17 (61%) | 0 |
| Treated with: steroids (S) | 14 (50%) | 0 |
| Treated with: gold (G) | 8 (29%) | 0 |
| Treated with: penicillamine (P) | 6 (21%) | 0 |
| Treated with: azathiprine (A) | 1 (4%) | 0 |
| Treated with: G/P/A | 8 (29%) | 0 |
| Treated with: S/G/P/A | 14 (50%) | 0 |

Rheumatoid factor profiles

Thirteen of the group of 33 (35%) were seropositive (titre≥1/40) on one or more occasion in the RAHA agglutination test for RF [*Med. Lab. Technol. 31:* 323—326 (1974)] and thus met the 8th ARA criterion for seropositivity. All these 13 developed classical/definite RA, and the frequency of seropositivity in the RA group was therefore 46%.

A total of 651 serum and synovial fluid samples were collected from the patients of the study and assayed by ELISA for IgM, RF, IgA RF and IgG RF as described above. With the results expressed as net absorbance, the IgA RF levels showed an overall highly significant association with IgM RF ($r=0.676$, $N=651$, $p<0.001$, Pearson's product-moment correlation method). Furthermore, the IgM RF levels showed a significant overall correlation with IgG RF ($r=0.407$, $N=643$, $p<0.001$, Pearson's product-moment correlation method).

Raised levels of IgM RF, IgA RF or IgG RF as measured by ELISA were observed at some stage in 19 (58%) of the group of 33. When the five non-RA patients were excluded, 18/28 (64%) developed raised RF at some stage. Table 6 shows the frequency of raised individual RF isotypes as measured by ELISA at first visit, 4.7±3.0 months (mean±1 SD) after the onset of symptoms. Seven (21%) and ten (30%) patients presented with raised IgA RF and IgM RF, respectively. After admission to the study only two patients developed raised IgA RF and three raised IgM RF.

Few extra-articular manifestations were seen in this group. One lady with persistent high serum level of IgA RF developed Felty's syndrome. Rheumatoid nodules were seen in only one patient who had raised IgM RF and IgA RF for normal IgG RF. One seronegative patient developed Sjoegren's syndrome.

TABLE 6
The frequency of raised RF levels in the prospective study group of 33,
both at onset and during the follow-up.

|  | Upper limit of normal* | No. with raised- at onset | No. with raised- at onset or later |
|---|---|---|---|
| IgA RF | <10 U/ml | 7 (21%) | 9 (27%) |
| IgM RF | ≤20 IU/ml | 10 (30%) | 13 (39%) |
| IgG RF | <80 U/ml | 4 (12%) | 8 (24%) |

\* Levels above the 95% upper limits for 102 normals, ≥10 U/ml, >20 IU/ml and ≥80 U/ml for IgA RF, IgM RF and IgG RF, respectively, were taken as raised.

Interrelations of clinical and serological parameters

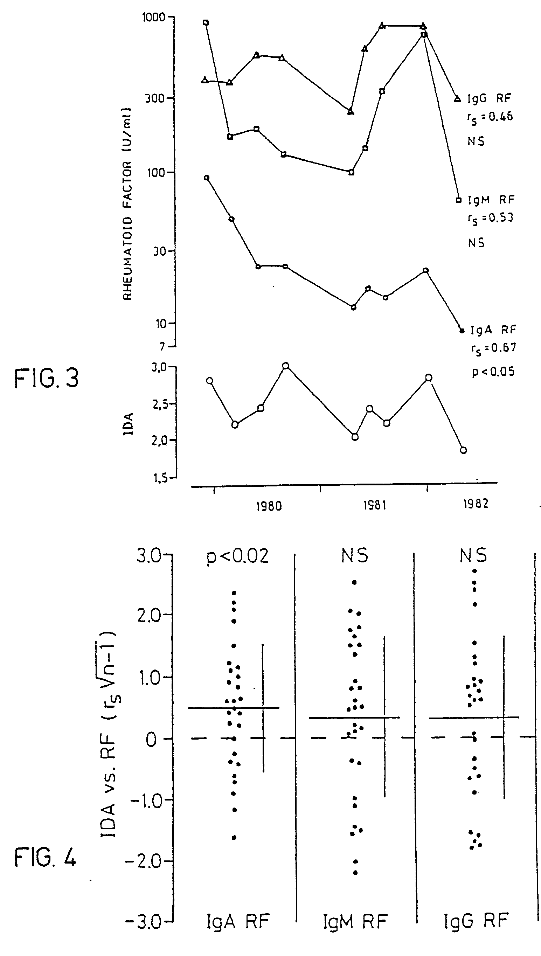

The follow-up profiles of EMS, PVAS, GS, ESR, IDA, IgM RF, IgA RF and IgG RF were intercorrelated for each of the 33 patients separately, using Spearman's rank correlation method. The number of assessments for each patient upon which the analysis was based varied from 5—12, mean 7.6. As an example, Figure 3 shows the follow-up profiles of IDA, IgM, RF, IgA RF and IgG RF for patient G. W. who developed classical RA. The rank correlation coefficients ($r_s$) for the association between the RF isotypes and the IDA were also shown in Figure 1. The association between the fluctuations in the IDA and the IgA RF was statistically significant ($r_s=0.67$, $p<0.05$).

Figure 4 shows the distribution in the group of 33 of the normalized Spearman's rank correlation coeficients ($r_s\sqrt{n-1}$) for the association between IDA and IgA, RF, IgM, RF and IgG RF, respectively. Although the scatter was considerable, the mean $r_s\sqrt{n-1}$ value for the association between IDA and IgA RF was significantly positive (a mean of 0.497 (SD 1.023), $p<0.02$ student's t test), implying an overall positive association in the group between fluctuations in IgA RF and disease activity during the follow-up. There was no such overall significant association, however, between fluctuations in IgM RF and IgG RF and IDA (Figure 4).

Table 7 shows the summarized results of a similar analysis where the within-patient association of fluctuations in EMS, PVAS, RAI, GS, ESR, IgA RF, IgG RF and IgM RF for the group of 33 was studied. The table shows the means ±1 SD of the $r_s\sqrt{n-1}$ values and the significance of the deviation from zero of the mean $r_s\sqrt{n-1}$. The fluctuations of the different clinical parameters for each patient were closely associated. For example, the interrelations of PVAS and RAI had a means $r_3\sqrt{n-1}$ of 0.96±1.00 (t=5.18, $p<0.001$). Similarly, the fluctuations of GS were closely associated with those of RAI and PVAS ($p<0.001$). The fluctuations in ESR closely followed those of GS (mean $r_s\sqrt{n-1}\pm1$ SD of −0.87±1.11, $p<0.001$). The fluctuations in ESR also showed a significant association with those of RAI (0.55±1.07, $p<0.05$) but there was not significant association between ESR and PVAS or EMS.

The fluctuations of the RF isotypes showed various interesting features. The fluctuations of IgA RF correlated closely with that of ESR (0.57±0.96, $p<0.01$), and there was also significant association between fluctuations in IgA RF and GS (−0.41±1.02, $p<0.05$), indicating that as GS deteriorated IgA RF tended to rise and vice versa.

The fluctuations of IgG RF also showed a significant correlation with those of ESR ($p<0.02$) and GS ($p<0.05$). However, there was no significant association between the fluctuations of IgG RF and IgA RF.

The fluctuations of IgM RF correlated significantly with those of IgA RF (0.60±1.22, $p<0.01$) and ESR ($p<0.05$) but not with fluctuations of the clinical parameters (Table 7).

Table 7 shows that the total number of individuals behind each means $r_s\sqrt{n-1}$ value was often less than 33. This was because no correlation coefficient could be calculated if a parameter remained constant throughout the observation period, for instance, four patients never had any early morning stiffness during the study. Consequently, the number behind each $r_s\sqrt{n-1}$ involving EMS never exceeded 29.

The relationship between the correlation coefficients ($r_s$) for parameters X vs. Y and the absolute values of X and Y during the study period was examined by a multivariate correlation analysis, employing Spearman's rank correlation method. The strength of the association was generally not related to the initial mean or maximal values of the parameters involved during the study period. For example, there was no significant correlation between the $r_s\sqrt{n-1}$ values for the association between IgA RF and IDA and the initial IgA RF values.

TABLE 7

The relationship between longitudinal fluctuations in clinical parameters, ESR and RSs during the follow-up of the group of 33.

| | | EMS | PVAS | RAI | GS | ESR | IgA RF | IgG RF |
|---|---|---|---|---|---|---|---|---|
| PVAS | N* | 27 | | | | | | |
| | $\overline{X}\pm1SD$** | 0.66±1.22 | | | | | | |
| | p<*** | 0.01 | | | | | | |
| RAI | N | 29 | 29 | | | | | |
| | $\overline{X}\pm1SD$ | 0.74±1.14 | ·0.96±1.00 | | | | | |
| | p< | 0.01 | 0.001 | | | | | |
| GS | N | 29 | 30 | 31 | | | | |
| | $\overline{X}\pm1SD$ | −0.56±0.96 | −0.65±0.95 | −0.75±0.96 | | | | |
| | p< | 0.01 | 0.001 | 0.001 | | | | |
| ESR | N | 29 | 31 | 30 | 32 | | | |
| | $\overline{X}\pm1SD$ | 0.38±1.04 | 0.20±1.07 | 0.55±1.26 | −0.87±1.11 | | | |
| | p< | NS | NS | 0.05 | 0.001 | | | |
| IgA RF | N | 28 | 31 | 29 | 31 | 32 | | |
| | $\overline{X}\pm1SD$ | 0.28±1.04 | 0.27±0.94 | −0.05±1.14 | −0.41±1.02 | 0.57±0.96 | | |
| | p< | NS | NS | NS | 0.05 | 0.01 | | |
| IgG RF | N | 28 | 28 | 30 | 30 | 31 | 30 | |
| | $\overline{X}\pm1SD$ | 0.33±1.12 | 0.25±1.26 | 0.24±1.30 | −0.05±1.25 | 0.51±1.12 | 0.03±1.13 | |
| | p< | NS | NS | NS | 0.05 | 0.02 | NS | |
| IgM RF | N | 29 | 30 | 31 | 32 | 33 | 32 | 31 |
| | $\overline{X}\pm1SD$ | 0.33±1.08 | 0.04±1.24 | 0.03±1.16 | −0.09±1.22 | 0.42±1.18 | 0.60±1.22 | 0.20±1.08 |
| | p< | NS | NS | NS | NS | 0.05 | 0.01 | NS |

\* The number of patients (≤33) for whom Spearman's rank correlation coefficients ($r_s$) could be calculated.
\*\* The mean and standard deviation of the normalised rank coefficients ($r_s\sqrt{n-1}$).
\*\*\* The significance of the deviation of $\overline{X}$ from zero as assessed by the student's t test.

EP 0 175 270 B1

## Discussion

The disease activity in the prospective group at the conclusion of the study, expressed as RAI, GS or IDA, correlated significantly with the number of erosions on the last available hand and wrist X-ray and it is well established that the occurrence of erosions in RA strongly indicates poor prognosis [*Amer. Rheum. Dis. 36*: 274—275 (1977)]. Consequently it seems valid to use the erosion count as objective evidence of outcome when assessing the prognostic value of RFs.

Our data indicate that an early rise in serum IgA RF during the course of RA is strongly predictive of a severe disease with early appearance of erosions (Figure 2). Apart from a recent preliminary communication from our group ["Rheumatoid factors (RF) in early rheumatoid arthritis (RA): A prospective study", A combined meeting of BARR, the Heberden Society, RSM Section of R&R and the Irish Society for R&R. 22—23 September 1983, University of Leeds, abstract No. 10], the question of whether IgA RF may be associated with prognosis has been associated with development of reactive arthritis [*J. Infect. Dis. 141*: 424—429 (1980)]. It is possible that the presence of IgA RF in severe RA reflects relatively active involvement of T cells in the pathogenic process because the generation of IgA antibodies is more dependent upon T cell help than is the production of IgM and possibly also IgG antibodies [*Immunol. Rev. 67*: 87—114 (1982)]. This hypothesis would agree with the view of Janossy et al [*Lancet 2*: 839—842 (1981)] that RA is characterised by an imbalance in T lymphocyte/macrophage immunoregulatory mechanisms with a relative dominance of T helper effects. IgA RF might therefore be a marker for heightened T helper activity in severe RA rather than pathogenic in itself.

We suggest that IgA RF may be a specific early marker for a poor prognosis in RA, perhaps justifying early treatment with specific drugs.

The natural course of RA is extremely variable, from the brief attack of synovitis which may barely meet conventional criteria for definite RA, to severe, destructive relentlessly progressive disease. It may therefore be expected that those patients who continue to attend a long-term follow-up clinic are preferentially those with the more severe disease. Indeed, some of those eight who were lost to follow-up probably failed to attend because they remained free of symptoms.

It is well established that the between-observer variation in assessements like the Ritchie articular index (RAI) is unacceptably high. As good technical reproducibility of the clinical and laboratory measures was of paramount importance in this study, the same physician carried out all the clinical asessments, and all the serum and synovial fluid samples for each patient were assayed for RFs (ELISA) on the same day. Furthermore, the clinic was always held at the same time of day thus reducing a possible diurnal variation.

Sixty-one per cent (17/28) of our RA patients developed erosive disease of their hands and wrists during the follow-up. That broadly agrees with Brook and Corbett [*Ann. Rheum. Dis. 36*: 71—73 (1977)] who found 43% (40/94) prevalence of "diagnostic" hand erosions after two years. As the range of the length of follow-up was relatively short (24—28 months) we felt justified in using the unmodified hand and wrist erosion count as a measure of the outcome.

The frequency of seropositivity in RA as judged by the sheep cell agglutination test is often quoted as being at least 75%. Consequently, the frequency of RAHA seropositivity in our group (39% of the whole group, 46% of the RA patients) may seem rather low. However, other authors have also noted low frequency of seropositivity in prospective studies of early RA. Masi et al [*Semin. Arthritis Rheum. 5*: 299—326 (1976)] studied 50 young adult RA patients, 38 of whom developed classical/definite RA, and found that only 44% (22/56) ever became seropositive. Our finding that only few patients developed raised RF level (ELISA) during the follow-up also agrees with Masi et al as only two of their 50 patients became sero-positive subsequent to the entry.

Several authors have attempted to correlate serum levels of rheumatoid factors and immune complexes with clinical features [*Arthritis Rheum. 26*: 593—598 (1983); *J. Rheumatol. 10*: 411—417 (1983); *Arthritis Rheum. 26* (Suppl. April 1983): 550 (1983)]. However, these calculations have mostly been based on cross-sectional analyses which fail to take into account individual differences in the baseline of parameters like for example the grip strength. Longitudinal within-patient studies would therefore seem more appropriate for the assessment of a possible relationship between for example RF levels and clinical indices of disease activity.

Our finding that of the three RF isotypes studies, IgG RF showed the closest association with fluctuations in disease activity, may seem unexpected. The data in Table 7 suggest that IgA RF may behave like an acute phase reactant. However, fluctuations in IgG RF also showed a significant association with both ESR and GS. It was interesting that even in patients with normal RFs, the RF fluctuations sometimes showed a significant association with fluctuations of the clinical parameters. These observations further strengthen our finding that the measurement of IgA RF may yield important information in RA as we showed that raised IgA RF in early RA was significantly associated with the later development of erosive disease whereas neither IgM RF nor IgG RF were.

The possible relation of IgA RF to disease activity and severity in RA has received limited attention, perhaps because IgA is a poor activator of complement and phagocytes and would therefore seem unlikely to be directly involved in the pathogenesis of RA. Marcolongo et al [*Ann. Rheum. Dis. 26*: 412—418 (1967)] reported that total serum IgA was raised in most RA patients but raised total serum IgG was only found in sero-positive RA. However, these workers did not find any relationship between the immunoglobulin levels

and the course or duration of disease. Furthermore, treatment of RA with gold or penicillamine may precipitate IgA deficiency although the mechanisms were unclear [*Ann. Rheum. Dis. 37*: 289 (1978)].

Recently, an interesting relationship between IgA and the HLA-B27-associated arthropathies has been reported. Ebringer [*Br. J. Rheumatol. 22* (Suppl. 2): 53—66 (1983)] showed that serum IgA levels in 122 ankylosing spondylitis (AS) patients were significantly related to their ESR and Wright et al [*Br. J. Rheumatol. 22* (Suppl. 2): 29—32 (1983)] suggested that IgA may behave like an acute phase reactant in patients with AS. Granfors et al [*J. Infect. Dis. 141*: 424—429 (1980)] furthermore showed that the persistence of IgA anti-yersinia antibodies was directly related to the development of arthritis following yersinia infection and Panayi [*Br. J. Rhematol. 22* (Suppl. 2): 119—121 (1983)] reported that serum anti-klebsiella IgA antibodies were significantly higher in patients with active AS than in patients with inactive AS, psoriatic arthritis or RA.

Lessard et al [*J. Rheumatol. 10*: 411—417 (1983)] recently reported that whereas IgG RF levels correlated both with articular index on one occasion and changes in articular index with time, IgA RF failed to show such a correlation. However, this was a cross-sectional study with only a few observations on each patient and over a relatively short period of time (a mean of 8.5 months).

It is not clear why IgA RF should be more closely associated with fluctuations in disease activity than the other RF isotypes. Perhaps a flare-up of RA is normally accompanied by polyisotypic RF production of which the IgG RF and IgM RF contribute to the inflammatory reaction by activation of complement and phagocytes. This in turn, may lead to rapid clearance and turnover of IgG RF and IgM RF in the joints and only a limited spillover into the peripheral circulation. IgA RF, however, being ineffective at activating complement and phagocytes, might persist longer in the body and thus serve as an easily detectable marker for disease activity in rheumatoid arthritis.

## Claims

1. A method for aiding the prognosis of individuals afflicted with rheumatoid arthritis, characterized in that:

(a) the level of the IgA isotype of rheumatoid factor (RF) in the blood of such individuals is measured, and

(b) such measured IgA RF level is compared with the level of normal individuals, whereby a substantially increased level of IgA RF in the tested individual indicates a likelihood that the disease will proceed to a severe state with the appearance of bone erosions.

2. The method of Claim 1 characterized in that the level of IgA is measured by immunoassay, preferably an enzyme-linked immunosorbent assay.

3. The method of Claim 2 characterized in that the assay comprises the steps of contacting a sample of serum obtained from the blood of the individual being tested with a solid phase comprising insolubilized normal rabbit IgG, after a predetermined incubation contacting the solid phase with the F(ab')$_2$ fraction of anti-human IgA antibody labelled with an enzyme, after a predetermined second incubation contacting the solid phase with the substrate for the enzyme which yields a detectable optical response, and measuring such response.

4. A method for aiding the prognosis of individuals afflicted with rheumatoid arthritis characterized in that:

(a) the level of the IgM isotype of rheumatoid factor (RF) in the blood of such individuals is measured, and

(b) such measured IgM RF level is compared with the level of normal individuals, whereby a substantially increased level of IgM RF in the tested individual indicates a likelihood that the disease will not proceed to a severe state.

5. The method of Claim 4 characterized in that the level of IgM RF is measured by immunoassay, preferably an enzyme-linked immunosorbent assay.

6. The method of Claim 5 characterized in that the assay comprises the steps of contacting a sample of serum obtained from the blood of the individual being tested with a solid phase comprising insolubilized normal rabbit IgG, after a predetermined incubation contacting the solid phase with anti-human IgM antibody labelled with an enzyme, after a predetermined second incubation contacting the solid phase with the substrate for the enzyme which yields a detectable optical response, and measuring such response.

7. A method for aiding the assessment of rheumatoid arthritis disease activity in individuals having the disease, characterized in that:

(a) the level of the IgA isotype of rheumatoid factor (RF) in the blood of such individual is measured, and

(b) such measured IgA RF level is compared with such levels measured over a prior period of time, whereby significant increase or decrease of such level with respect to the prior measured levels indicates an increase or decrease, respectively, in disease activity.

8. The method of Claim 7 wherein the level of IgA RF is measured by immunoassay, preferably an enzyme-linked immunosorbent assay.

# EP 0 175 270 B1

**Patentansprüche**

1. Verfahren zur Unterstützung der Prognose für Individuen, die an rheumatischer Arthritis leiden, dadurch gekennzeichnet, daß

(a) der Pegel des IgA-Isotyps des Rheumafaktors (RF) im Blut dieser Individuen gemessen wird und

(b) der so gemessene IgA-RF-Pegel verglichen wird mit dem Pegel normaler Individuen, wobei ein wesentlich erhöhter Pegel von IgA-RF bei dem getesteten Individuum eine Wahrscheinlichkeit anzeigt, daß die Krankheit zu einem ernsten Zustand mit dem Auftreten von Knochenerosionen fortschreitet.

2. Verfahren nach Anspruch 1, dadurch gekennzeichnet, daß der IgA-Pegel gemessen wird durch Immunoassay, vorzugsweise Enzyme Linked Immunosorbent-Assay (ELISA).

3. Verfahren nach Anspruch 2, dadurch gekennzeichnet, daß der Assay die Schritte umfaßt, daß man eine Serumprobe, die von dem Blut des zu testenden Individuums erhalten wurde, mit einer Festphase in Kontakt bringt, die unlösliches normales Kaninchen-IgG enthält, nach einer vorbestimmten Inkubation die Festphase mit der F(ab')$_2$-Fraktion von Anti-Human-IgA-Antikörper, der mit einem Enzym markiert ist, in Kontakt bringt, nach einer vorbestimmten zweiten Inkubation die Festphase mit dem Substrat für das Enzym in Kontakt bringt, das eine nachweisbare optische Antwort ergibt und die Antwort mißt.

4. Verfahren zur Unterstützung der Prognose für Individuen, die an rheumatischer Arthritis leiden, dadurch gekennzeichnet, daß

(a) der Pegel von IgM-Isotyp des Rheumafaktors (RF) im Blut dieser Individuen gemessen wird und

(b) der so gemessene IgM-RF-Pegel verglichen wird mit dem Pegel normaler Individuen, wobei ein wesentlich erhöhter Pegel an IgM-RF bei dem getesteten Individuum eine Wahrscheinlichkeit anzeigt, daß die Krankheit nicht in einen ernsten Zustand forschreitet.

5. Verfahren nach Anspruch 4, dadurch gekennzeichnet, daß der Pegel an IgM-RF durch Immunoassay, vorzugsweise ELISA, gemessen wird.

6. Verfahren nach Anspruch 5, dadurch gekennzeichnet, daß der Assay die Schritte umfaßt, daß man eine Serumprobe, die aus dem Blut des zu testenden Individuums erhalten wurde, mit einer festen Phase in Kontakt bringt, die unlösliches normales Kaninchen-IgG enthält, nach einer vorbestimmten Inkubation die Festphase mit Anti-human-IgM-Antikörper, der mit einem Enzym markiert ist, in Kontakt bringt, nach einer vorbestimmten zweiten Inkubation die Festphase mit dem Substrat für das Enzym in Kontakt bringt, was eine nachweisbare optische Antwort ergibt und diese Antwort mißt.

7. Verfahren zur Unterstützung der Feststellung von Rheumaarthritis-Krankheitsaktivität in Individuen, die diese Krankheit haben, dadurch gekennzeichnet, daß

(a) der Pegel des IgA-Isotyps von Rheumafaktor (RF) im Blut dieser Individuen gemessen wird und

(b) der so gemessen IgA-RF-Pegel verglichen wird mit den Pegeln, die über einen früheren Zeitraum gemessen, wurden, wobei ein signifikanter Anstieg oder Abfall des Pegels im Hinblick auf die früher gemessenen Pegel ein Anwachsen bzw. Abnehmen der Krankheitsaktivität anzeigt.

8. Verfahren nach Anspruch 7, worin der Pegel von IgA-RF gemessen wird durch Immunoassay, vorzugsweise ELISA.

**Revendications**

1. Procédé pour faciliter le pronostic de patients atteints d'arthrite rhumatoïde caractéristé en ce qu l'on mesure:

(a) le niveau d'isotype de l'IgA du facteur rhumatoïde (FR) dans le sang de ces patients et

(b) que ce niveau du facteur rhumatoïde IgA mesuré est comparé à celui de personnes normales, grâce à quoi un niveau sensiblement accru du FR IgA de la personne testée indique une probabilité d'évolution de la maladie vers un état grave, avec apparition d'érosion osseuse.

2. Procédé selon la revendication 1, caractérisé en ce que le niveau d'IgA est mesuré par essai immunologique, de préférence par essai d'immunosorption à liaison enzymatique.

3. Procédé selon la revendication 2, caractérisé en ce que l'essai comprend les phases de mise en contact d'un échantillon de sérum tiré du sang de la personne testée, avec une phase solide comprenant des IgG de lapin normal insolubilisés, après une incubation prédéterminée de contact de la phase solide avec la fraction F(ab')$_2$ de l'anticorps de l'anti-IgA humain à marquage enzymatique, après une seconde incubation prédéterminée de contact de la phase solide avec le substrat enzymatique donnant une réponse optique détectable, et la mesure de cette réponse.

4. Procédé pour aider au pronostic de patients atteints d'arthrite rhumatoïde, caractérisé en ce que:

(a) l'on mesure le niveau d'isotype IgM du facteur rhumatoïde (FR) dans le sang de ces patients et

(b) que ce niveau de FR IgM mesuré est comparé au niveau de personnes normales, grâce à quoi un niveau sensiblement accru de FR IgM chez la personne testée indique une probabilité que la maladie ne progressera pas vers un état grave.

5. Procédé selon la revendication 4, caractérisé en ce que le niveau de FR IgM est mesuré par essai immunologique, de préférence un essai d'immunosorption à liaison enzymatique.

6. Procédé selon la revendication 5, caractérisé en ce que l'analyse comprend les phases de mise en contact d'un échantillon de sérum tiré du sang de la personne testée avec une phase solide comprenant des IgG de lapin normal insolubilisés, après une incubation prédéterminée de contact de la phase solide

**EP 0 175 270 B1**

d'un anticorps d'anti-IgM humain à marquage enzymatique, après une seconde incubation prédéterminée de contact de la phase solide avec le substrat enzymatique donnant une réponse optique détectable, et la mesure de cette réponse.

7. Procédé pour faciliter l'évaluation de l'activité de la maladie de l'arthrite rhumatoïde chez des patients atteints de la maladie, caractérisé:

(a) en ce que l'on mesure le niveau d'isotype IgA du facteur rhumatoïde (FR) dans le sang de ces patients et

(b) que ce niveau FR IgA mesuré est comparé à des niveau mesurés sur une période du temps antérieure, grâce à quoi une augmentation ou une réduction significative de ce niveau par rapport aux niveaux mesurés antérieurement indique, respectivement, une augmentation ou une aggravation de l'activité de la maladie.

8. Procédé selon la revendication 7, dans lequel le niveau de FR IgA est mesuré par essai immunologique, de préférence par un essai d'immunosorption à liaison enzymatique.

FIG. 1

EP 0 175 270 B1

FIG. 2

EP 0 175 270 B1

FIG. 3

FIG. 4